# DEMANDE DE BREVET EUROPEEN

(11) **EP 0 887 044 A1**
(43) Date de publication de la demande: **30.12.1998**
(21) Numéro de dépôt: 97450014.2
(22) Date de dépôt: 26.06.1997
(51) Int. Cl.: A61B 5/113, G08B 21/00

(54) **Dispositif de surveillance des arrêts respiratoires plus particulièrement pour les nouveaux-nés**

(71) Demandeur: Babidor (S.à.r.l.), 40110 Arengosse (FR)
(72) Inventeur: Courbi, Gérard, 40110 Mont-de-Marsan (FR); Calmettes, Guy, 40090 Saint Martin D'Oney (FR)
(74) Mandataire: Thébault, Jean-Louis

(57) **Abrégé**

L'invention concerne un dispositif de surveillance des arrêts respiratoires, plus particulièrement pour un nouveau-né (10) dormant dans un lit (12), prévu pour être disposé sur le sommier du lit, sous le matelas, comprenant deux plaques inférieure (16) et supérieure (24) avec un capteur (18) sensible à la pression, monté en sandwich entre lesdites plaques, des moyens (14) de contrôle et d'alarme prévus pour réagir dès que le capteur (18) reste sans émettre de signal pendant une période donnée, caractérisé en ce que les plaques sont solidarisées l'une à l'autre par des plots collés (26) sur leurs faces en vis à vis des plaques (16,24), disposés sensiblement à la périphérie de ces plaques.

## Description

La présente invention a pour objet un dispositif de surveillance des arrêts respiratoires, notamment des apnées du sommeil des nourrissons.

On connaît des systèmes qui ont pour but de surveiller les apnées du sommeil, notamment celui qui est exposé dans la demande de brevet anglais GB-A-2 192 460.

Le dispositif comprend deux plaques superposées, plus spécifiquement identiques en dimensions pour permettre une bonne superposition, entre lesquelles est disposé un capteur piezoélectrique qui assure la fonction de détection des variations de la pression exercée sur les plaques. Ce capteur est fixé sur l'une des plaques, en l'occurrence la plaque supérieure.

Cette plaque supérieure, prévue pour être disposée sous le corps du nouveau-né, est semi-rigide, tandis que la plaque inférieure est plus souple.

Une enveloppe est rapportée autour de l'ensemble en sorte de maintenir les deux plaques en position strictement superposée, c'est à dire en évitant les déplacements relatifs en cisaillement d'une plaque par rapport à l'autre.

Le capteur est relié à une alarme par un circuit de traitement de l'information qui génère un signal acoustique, vocal ou lumineux, lorsque le capteur reste sans sollicitation pendant une durée prédéterminée, susceptible de correspondre à un arrêt respiratoire du nouveau-né.

Cette demande de brevet correspond strictement à l'art antérieur à partir duquel le demandeur a étudié le perfectionnement objet de la présente invention décrite ultérieurement.

Une autre demande de brevet est intéressante par rapport à ce perfectionnement, EP-A-514 744.

Dans cette demande, les éléments constructifs précédemment exposés dans la demande de brevet anglais sont repris mais avec un agencement différent. Le mode de réalisation prévoit une plaque surmontée d'un capteur, ladite plaque étant elle-même supportée par un anneau. L'ensemble est immobilisé dans une enveloppe en interposant une cale entre le capteur et ladite enveloppe.

La cale située au-dessus du capteur et l'anneau peuvent venir de fabrication avec ladite enveloppe.

On peut constater que dans ce cas, l'enveloppe joue un rôle de maintien en position des plaques ce qui oblige à placer l'enveloppe au plus près par rapport au capteur et aux plaques. Ceci peut conduire à des diminutions de sensibilité si l'enveloppe est trop plaquée et à des mouvements des plaques préjudiciables à la bonne tenue mécanique de l'enveloppe dans le temps si l'enveloppe est trop ample. De plus, beaucoup plus grave, une enveloppe trop ample peut générer des plis qui sont susceptibles de se loger entre les plaques, bloquant ou tout le moins entravant le bon fonctionnement du dispositif.

De plus, lorsque l'on souhaite changer l'enveloppe pour des raisons d'hygiène ou de vieillissement, par exemple en milieu hospitalier, le démontage n'est pas aisé par un profane, simple utilisateur car le retrait de l'enveloppe provoque le déplacement des plaques et le remontage est difficile avec des risques importants de non-fonctionnement, or un tel dispositif est amené à jouer son rôle d'alerte de façon exceptionnelle mais par contre, il faut que le moment où cela s'avère nécessaire, le dispositif remplisse son rôle.

Le but de la présente invention est de proposer un dispositif de surveillance de fabrication simple, d'une très grande sensibilité, d'une très grande fiabilité, d'une grande reproductibilité, dont le montage ne laisse aucune possibilité d'erreur, dont l'enveloppe a pour seul rôle la protection du capteur et des plaques et dont le prix de revient ne subit aucun surcoût.

A cet effet, le dispositif de surveillance des arrêts respiratoires selon l'invention, plus particulièrement pour un nouveau-né dormant dans un lit, prévu pour être disposé sur le sommier du lit, sous le matelas, comprend deux plaques inférieure et supérieure avec un capteur sensible à la pression, monté en sandwich entre lesdites plaques, des moyens de contrôle et d'alarme prévus pour réagir dès que le capteur reste sans émettre de signal pendant une période donnée, caractérisé en ce que les plaques sont solidarisées l'une à l'autre par des plots collés sur leurs faces en vis à vis des plaques, disposés sensiblement à la périphérie de ces plaques.

Plus particulièrement selon un mode de réalisation préférentiel, les plots sont en mousse avec double-faces autocollantes, d'une hauteur sensiblement égale à celle du capteur, en sorte d'éviter toute mise en compression du capteur, lesdits plots en mousse travaillant avec des faibles taux de compression et d'extension.

Selon un agencement particulier, les plaques sont carrées et de mêmes dimensions et les plots sont disposés aux quatre angles des plaques.

Selon une autre caractéristique, il est prévu un amortisseur interposé entre le capteur et la plaque supérieure.

Cette plaque inférieure est d'une grande rigidité.

Le dispositif comprend en outre une enveloppe de protection de l'ensemble des plaques et du capteur.

Ce capteur de pression est un capteur piezoélectrique et les plaques sont en fibres végétales comprimées.

La présente invention est décrite ci-après selon un agencement particulier, non limitatif, en regard des dessins annexés qui représentent :
- figure 1, une vue d'une installation du dispositif de détection des apnées du sommeil selon l'invention,
- figure 2, une vue en perspective du dispositif isolé,
- figure 3, une vue en coupe médiane par un plan vertical du dispositif de la figure 2, et
- figure 4A et 4B, une représentation respectivement du fonctionnement d'un dispositif de l'art antérieur, dans le cas où les plaques sont maintenues par une enveloppe et un dispositif selon l'invention, avec les plaques maintenues par des plots.

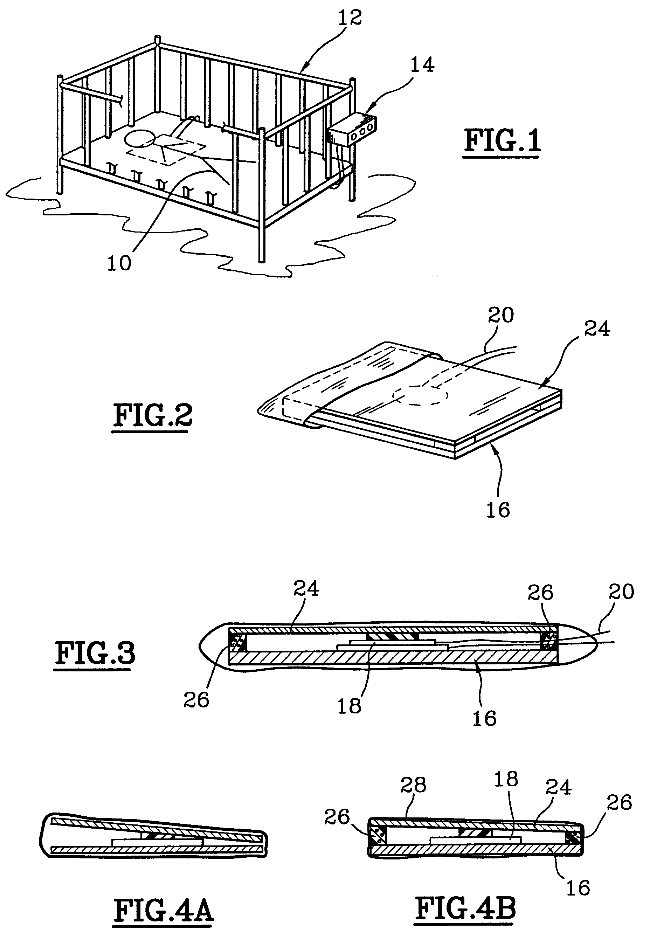

Sur la figure 1, on a représenté un dispositif en trait discontinu placé sous un nouveau-né 10, dans un lit pour enfant 12. Le dispositif est relié de façon connue à des moyens de contrôle et d'alarme 14. De tels moyens comprennent généralement un récepteur de signal, un amplificateur de signal, un comparateur de réception d'un signal avec une base temps pré-programmée et des moyens d'alarme visuelle, sonore, acoustique. Un tel circuit peut être réalisé de différentes manières et il ne relève pas de la présente invention dont il n'est que le complément. Son but est de déclencher les moyens d'alarme lorsque le capteur n'enregistre aucun signal pendant une durée donnée, de l'ordre de 10 à 30 secondes pour donner un ordre de grandeur.

Sur la figure 2, on a représenté une plaque inférieure 16, d'une grande rigidité sur laquelle est solidarisé un capteur piezoélectrique 18, dont les fils de connexion 20 sont reliés aux moyens de contrôle et d'alarme 14 par exemple en matériau vendu dans le commerce sous la dénomination "isorel", en épaisseur 5 mm.

Une seconde plaque 24 est superposée à la première en sorte de prendre en sandwich le capteur piezoélectrique 18, avec interposition d'un amortisseur 25 pour éviter que les chocs éventuels puissent dégrader ledit capteur.

Cette seconde plaque est d'une moins grande rigidité, par exemple également en matériau vendu dans le commerce sous la dénomination "isorel", en épaisseur 2 mm.

Ces deux plaques sont ensuite liées entre elles par des plots 26 disposés préférentiellement dans les angles des plaques. Ces plots sont en un matériau à mousse double-faces autocollantes.

Ainsi lors du montage, les plots sont tout d'abord collés sur la plaque inférieure et la plaque supérieure est rapportée par dessus et vient se coller sur les plots provoquant la solidarisation définitive des deux plaques et emprisonnant le capteur sans exercer de pression pour autant que les plots aient été choisis de hauteur convenable.

Le choix d'un tel mode de réalisation apporte un progrès considérable qui s'explique.

En effet, un telle liaison évite les mises en porte à faux de l'une des plaques par rapport à l'autre. La sensibilité de déclenchement peut être réglée de façon plus fine car les déplacements parasites liés à des mouvements intempestifs de l'une des plaques par rapport à l'autre sont contrôlés par les plots.

Lors d'un mouvement, si l'un des côtés de la plaque est soumis à un effort, la pression sur le capteur est amplifiée du fait que le côté opposé est retenu par les plots si bien qu'il y a un effet de levier. Ainsi sur la figure 4A, on remarque que l'effort exercé sur un côté soulève la plaque qui n'est retenue que par l'enveloppe, tandis que dans le cas de la figure 4B, la plaque est maintenue sur le côté opposé si bien que l'effort exercé sur le capteur est amplifié, l'enveloppe ne jouant aucun rôle.

On constate que l'enveloppe 28 n'a pas été mentionnée parce qu'elle n'intervient pas pour la mise en oeuvre du dispositif. Elle est, bien entendu, nécessaire pour la protection du dispositif vis à vis des liquides comme les écoulements urinaires ou de tout autre produit notamment en milieu médical, mais il s'agit d'une simple housse. On veillera par contre à ce qu'elle ne forme aucun pli susceptible de venir entraver le bon fonctionnement du capteur, mais cette contrainte est compensée par le fait que l'enveloppe peut être très mince, donc très souple et donc montée au plus juste par rapport aux plaques.

On peut remarquer l'adaptation particulière de ce type de dispositif pour les matelas reposant sur un sommier à lattes car la plaque inférieure de grande rigidité peut rester en appui sur deux ou trois lattes, sans fléchir, tandis que la plaque supérieure reste sensible, en appui sur les plots amortisseurs.

Les plaques peuvent être également de nature autre que des fibres végétales comprimées et peuvent être réalisées en matière plastique, en bois, en aluminium.

## Revendications

1. Dispositif de surveillance des arrêts respiratoires, plus particulièrement pour un nouveau-né (10) dormant dans un lit (12), prévu pour être disposé sur le sommier du lit, sous le matelas, comprenant deux plaques inférieure (16) et supérieure (24) avec un capteur (18) sensible à la pression, monté en sandwich entre lesdites plaques, des moyens (14) de contrôle et d'alarme prévus pour réagir dès que le capteur reste sans émettre de signal pendant une période donnée, caractérisé en ce que les plaques (16, 24) sont solidarisées l'une à l'autre par des plots (26) collés sur leurs faces en vis à vis des plaques, disposés sensiblement à la périphérie de ces plaques.

2. Dispositif de surveillance des arrêts respiratoires selon la revendication 1, caractérisé en ce que les plots (26) sont en mousse avec double-faces autocollantes, d'une hauteur sensiblement égale à celle du capteur (18), en sorte d'éviter toute mise en compression du capteur, lesdits plots en mousse travaillant avec des faibles taux de compression et d'extension.

3. Dispositif de surveillance des arrêts respiratoires selon la revendication 1 ou 2, caractérisé en ce que les plaques (16,24) sont carrées et de mêmes dimensions et les plots (26) sont disposés aux quatre angles des plaques.

4. Dispositif de surveillance des arrêts respiratoires selon l'une quelconque des revendications précédentes, caractérisé en ce qu'il comprend un amortisseur (25) interposé entre le capteur et la plaque supérieure.

5. Dispositif de surveillance des arrêts respiratoires selon l'une quelconque des revendications précédentes, caractérisé en ce que la plaque inférieure (16) est d'une grande rigidité.

6. Dispositif de surveillance des arrêts respiratoires selon l'une quelconque des revendications précédentes, caractérisé en ce qu'il comprend une enveloppe (28) de protection de l'ensemble des plaques et du capteur.

7. Dispositif de surveillance des arrêts respiratoires selon l'une quelconque des revendications précédentes, caractérisé en ce que le capteur (18) de pression est un capteur piezoélectrique et les plaques sont en fibres végétales comprimées.
